(19) Europäisches Patentamt
European Patent Office
Office européen des brevets

(11) **EP 2 735 615 A1**

(12) **EUROPEAN PATENT APPLICATION**
published in accordance with Art. 153(4) EPC

(43) Date of publication:
**28.05.2014 Bulletin 2014/22**

(21) Application number: **12818183.1**

(22) Date of filing: **20.07.2012**

(51) Int Cl.:
*C12P 7/40* [(2006.01)]  *C07C 51/487* [(2006.01)]
*C07C 59/08* [(2006.01)]  *C08G 63/78* [(2006.01)]

(86) International application number:
**PCT/JP2012/068427**

(87) International publication number:
**WO 2013/015212 (31.01.2013 Gazette 2013/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.07.2011 JP 2011160643**

(71) Applicant: **Toray Industries, Inc.
Tokyo, 103-8666 (JP)**

(72) Inventors:
• **YAMADA, Tetsuya
  Kanagawa 2488555 (JP)**

• **ITO, Masateru
  Kanagawa 2488555 (JP)**
• **YAMADA, Katsushige
  Kanagawa 2488555 (JP)**
• **KAWAMURA, Kenji
  Kanagawa 2488555 (JP)**

(74) Representative: **Kador & Partner
Corneliusstrasse 15
80469 München (DE)**

(54) **METHOD FOR PRODUCING ORGANIC ACID**

(57) By subjecting an organic acid derived from a biomass resource to oxidation treatment using an oxidizing agent such as hydrogen peroxide, tert-butylhydroperoxide, ozone, sodium hypochlorite or sodium chlorite, colored impurities contained in the organic acid derived from a biomass resource can be removed.

EP 2 735 615 A1

**Description**

TECHNICAL FIELD

The present invention relates to a method for producing an organic acid derived from a biomass resource.

BACKGROUND ART

Due to the growing concern over a rise in the prices of petroleum resources and their depletion, recent interest has focused on production of polymer materials using biomass resources, which are renewable resources, as raw materials. In particular, organic acids, which can be used as raw materials for polyester and the like, are drawing attention. Examples of the method for producing such biomass resource-derived organic acids include methods in which an organic acid is obtained by direct chemical synthesis from a biomass-derived compound, and methods in which an organic acid is obtained by fermentation culture of a microorganism with a biomass-derived compound. However, in these methods, the produced organic acid needs to be processed by a combination of laborious purification steps such as crystallization, membrane separation and/or distillation in order to remove many kinds of impurities derived from the biomass resource, and, especially among such impurities, colored impurities, even in very small amounts, cause coloration of the polymer in a later step. Polymers obtained using colored organic acids are also colored, and this may lead to not only low commercial values but also low physical properties due to the influence of small amounts of causative agents of the coloration contained in the polymers.

As a method for removing colored impurities contained in a biomass resource-derived organic acid, a processing method using a reducing agent for an aqueous lactic acid solution containing pyruvic acid obtained by fermentation is known (Patent Document 1). It has been suggested that, by this method, a lactic acid solution that does not contain pyruvic acid and is hardly colored can be obtained, and that a high-molecular-weight polylactic acid can be obtained using the solution.

PRIOR ART DOCUMENTS

[Patent Document]

[Patent Document 1] JP 3880175 B

SUMMARY OF THE INVENTION

PROBLEMS TO BE SOLVED BY THE INVENTION

The present inventors discovered that, in cases where colored impurities (for example, pyruvic acid) contained in biomass resource-derived lactic acid are removed by reduction treatment, which is a conventional method, racemic lactic acid is produced as the reduction treatment of pyruvic acid proceeds. This is problematic since there is a concern of a low optical purity of the obtained lactic acid and a low melting point of the polylactic acid obtained by polymerization of the lactic acid. In view of this, the present invention aims to provide a processing method that allows, without influencing physical properties of organic acids derived from biomass resources, more efficient reduction in colored impurities.

MEANS FOR SOLVING THE PROBLEMS

As a result of intensive study to solve the above problem, the present inventors focused attention on oxidizing agents, which are used as reaction reagents in organic synthesis reactions. The present inventors then discovered that oxidation treatment of an organic acid derived from a biomass resource containing colored impurities enables reduction in the degree of coloration without deteriorating the physical properties of the organic acid, thereby completing the present invention.

That is, the present invention is constituted by (1) to (7) below.

(1) A method for producing an organic acid, the method comprising subjecting an organic acid derived from a biomass resource to oxidation treatment using an oxidizing agent.
(2) The method for producing an organic acid according to (1), wherein the oxidizing agent is one or more selected from the group consisting of hydrogen peroxide, tert-butylhydroperoxide, ozone, sodium hypochlorite and sodium chlorite, and aqueous solutions thereof.
(3) The method for producing an organic acid according to (1) or (2), wherein the oxidation treatment is combined with heat treatment.

(4) The method for producing an organic acid according to (3), wherein the heat treatment is distillation.

(5) The method for producing an organic acid according to any one of (1) to (4), wherein the organic acid is an organic acid obtained by fermentation culture of a microorganism(s).

(6) The method for producing an organic acid according to any one of (1) to (5), wherein the organic acid is one or more selected from the group consisting of lactic acid, hydroxybutyric acid, 3-hydroxypropionic acid, itaconic acid, glycolic acid, adipic acid, muconic acid, acrylic acid, succinic acid, sebacic acid, 2,5-furandicarboxylic acid and terephthalic acid.

(7) A method for producing an organic acid polymer, the method comprising polymerizing as a raw material an organic acid obtained by the method for producing an organic acid according to any one of (1) to (6).

EFFECT OF THE INVENTION

By the present invention, a high-purity organic acid with a lower degree of coloration, derived from a biomass resource, can be obtained. Further, since organic acids obtained by the present invention do not suffer from deterioration of their physical properties by the process of removal of colored impurities, polymers produced by polymerization of the organic acids have better properties in, for example, color, the weight average molecular weight, the melting point and the weight reduction rate upon heating, compared to polymers produced by polymerization of organic acids obtained by conventional methods.

BEST MODE FOR CARRYING OUT THE INVENTION

Specific examples of the organic acid derived from a biomass resource (hereinafter simply referred to as organic acid) include products produced by fermentation culture of microorganisms capable of utilizing raw materials derived from biomass resources such as polysaccharides including cellulose, and monosaccharides including glucose and xylose; products produced from biomass resource-derived raw materials by known synthesis/degradation reactions; and products produced by combination of these methods. In the present invention, production of an organic acid obtained by fermentation culture of a microorganism capable of utilizing a biomass resource is especially preferred. The organic acid as the subject of the present invention is not limited, and the subject is preferably a polymer material (monomers) for which there is a concern of deterioration of the quality due to colored components derived from a biomass resource. Examples of the organic acid as the polymer material include lactic acid, hydroxybutyric acid, 3-hydroxypropionic acid, itaconic acid, glycolic acid, adipic acid, muconic acid, acrylic acid, succinic acid, sebacic acid, 2,5-furandicarboxylic acid and terephthalic acid, and one or more of these may be used as the subject.

Examples of the method for producing an organic acid by fermentation culture of a microorganism capable of utilizing a biomass resource include the method described in JP 2008-029329 A, in which lactic acid is produced by fermentation culture of a microorganism; the method described in JP 6-38775 A, in which 3-hydroxybutyric acid is produced by fermentation culture of a microorganism; the method described in JP 4490628 B, in which 3-hydroxypropionic acid is produced by fermentation culture of a microorganism; the method described in JP 1913914 B, in which itaconic acid is produced by fermentation culture of a microorganism; the method described in JP 2008-56654 A, in which glycolic acid is produced by fermentation culture of a microorganism; the method described in JP 4380654 B, in which succinic acid is produced by fermentation culture of a microorganism; the method in which, as described in Enzyme and Microbial Technology, 27, 205(2000), sebacic acid is produced by a microorganism having a capacity to produce sebacic acid using decanoic acid as a raw material; and the method described in WO2011/094131, in which terephthalic acid is produced by fermentation culture of a microorganism.

Examples of the method for producing an organic acid from a biomass resource by combination of known chemical synthesis reactions include the method described in JP 2011-84540 A, in which glucose or fructose is converted to 5-hydroxymethylfurfural by acid treatment or the like, and this is followed by oxidation to produce 2,5-furandicarboxylic acid, which can be used as a raw material for polyesters. Examples of the method for producing an organic acid by thermal decomposition of a biomass include, as described in Science, 330, 1222(2010), a method in which a cellulose biomass such as wood waste is subjected to heat pressurization treatment under hydrogen atmosphere to obtain xylene, and this is followed by oxidation by a known method to produce terephthalic acid, which can be used as a raw material for polyesters.

Examples of the method for producing an organic acid by subjecting a product obtained by fermentation culture of a microorganism capable of utilizing a biomass resource to chemical synthesis reaction include the method described in US 5487987 B, in which muconic acid is produced by fermentation culture of a microorganism having a capacity of muconic acid fermentation, and the product is then subjected to hydrogen reduction to produce adipic acid, which can be used as a raw material for nylons; and the method described in JP 4490628 B, in which 3-hydroxypropionic acid is produced by fermentation culture of a microorganism having a capacity of muconic acid fermentation, and the product is then subjected to dehydration reaction to produce acrylic acid.

Although it is known that colored impurities are contained in the organic acids obtained by the methods described above, the present inventors discovered that the colored impurities are decomposed by oxidation reaction with an oxidizing agent, thereby completing the present invention. The details of the process are described below.

The oxidizing agent used in the present invention is not limited, and oxidizing agents used in general chemical reactions can be used. Preferred examples of the oxidizing agent include hydrogen peroxide and aqueous solutions thereof, sodium hypochlorite and aqueous solutions thereof, sodium chlorite, tert-butylhydroperoxide and aqueous solutions thereof, ozone and aqueous solutions thereof, oxygen, 2-iodoxybenzoic acid, manganese dioxide, Dess-Martin periodinane and 2,3-dicyano-5,6-dichloro-1,4-benzoquinone. More preferred examples of the oxidizing agent include hydrogen peroxide and aqueous solutions thereof, sodium hypochlorite and aqueous solutions thereof, sodium chlorite, tert-butylhydroperoxide and aqueous solutions thereof, and ozone and aqueous solutions thereof. Each of these oxidizing agents may be used alone, or two or more of these may be used.

When the colored impurities contained in the organic acid is subjected to oxidation treatment using an oxidizing agent, the organic acid is preferably in the state where the organic acid is dissolved in a solvent, but may also be in the state where the organic acid containing colored substances derived from a biomass is not completely dissolved in a solvent, or in the state of a slurry. Either an aqueous or organic solvent system may be applied as the solvent for the organic acid, and an aqueous solvent is preferably employed.

The method for adding the oxidizing agent to the organic acid is not limited. In cases where the oxidizing agent is a solid or liquid, the agent may be added to the organic acid directly or as a solution, and, in cases where the oxidizing agent is in the form of a gas, the agent may be added by a method in which the agent is directly blown into an organic acid solution, or by a method in which the oxidizing agent in the form of a gas is first dissolved in water or dispersed in water as microbubbles, and then fed to an organic acid solution.

Further, in order to rapidly obtain the effect of reducing colored components, heat treatment is preferably carried out in combination. The heat treatment herein means heating at a temperature of not less than 35°C, and the temperature is preferably 35 to 200°C, more preferably 50 to 180°C, still more preferably 60 to 180°C. The heat treatment may be carried out before the oxidation treatment, or the oxidation treatment may be carried out under heat.

In cases where the heat treatment is carried out before the oxidation treatment, the degree of coloration of the reaction solution (liquid to be treated) increases, but the degree of coloration decreases by the oxidation treatment of the solution.

In cases where the oxidation treatment is carried out under heat, the method is not limited to methods by simply heating the reaction solution. For example, in cases where distillation is carried out in a later step, the heating operation in the distillation operation may be regarded as the heat treatment. That is, in such cases, distillation of the reaction solution is carried out in the presence of the oxidizing agent. Since, in this method, the oxidation treatment and the heat treatment, in addition to isolation of the organic acid with a decreased degree of coloration, can be simultaneously carried out, the method is preferably applied in the present invention.

The oxidation treatment in the present invention may be combined with another purification operation. Depending on the type of the oxidizing agent employed, the agent may be dissolved or precipitated in the solution after reaction with coloring substances. The method for isolating the substances from the organic acid of interest is not limited, and examples of the method include ion exchange, filtration and distillation. In particular, in cases where the purification operation to be carried out in combination is distillation, the operation can be carried out simultaneously with the heat treatment, which is efficient and hence preferred.

The oxidizing agent treatment is finished when the color no longer changes. The length of time required for the color to stop changing varies depending on the type of the oxidizing agent employed and the amount of the agent added.

Whether or not the colored components derived from a biomass, contained in the organic acid, were reduced by the present invention is evaluated by measuring the color of the organic acid before the oxidation treatment and after the oxidation treatment in terms of the APHA unit color number (JIS K 0071-1, established on October 20, 1998; hereinafter referred to as the APHA value). That is, when the treatment was carried out under the same conditions except for the oxidation treatment, in cases where the APHA value after the oxidation treatment is lower than the APHA value before the oxidation treatment, it is judged that the effect of the present invention was obtained.

Using the organic acid obtained by the present invention, an organic acid polymer can be produced by a known polymerization method. The organic acid polymer means a polymer produced by polymerization using an organic acid as monomers. Specific examples of the organic acid polymer include the organic acid polyesters and organic acid polyamides described below.

[Organic Acid Polyesters]

For example, in cases of a bifunctional oxycarboxylic acid containing a hydroxyl group in the molecule, such as lactic acid, glycolic acid or hydroxybutyric acid, it may be polymerized alone to obtain a polyester. Examples of the polymerization method for production of a polyester include a two-step polymerization method in which a cyclic dimer such as lactide in the cases of polylactic acid, or glycolide in the cases of glycolic acid, is first produced, and ring-opening polymerization is then carried out; and a single-step direct polymerization method in which the organic acid is directly subjected to dehydration polycondensation in a solvent or under solvent-free conditions. Specific examples of the polyester include polylactic acid, polyglycolic acid, polyhydroxypropionic acid and polyhydroxybutyric acid.

Further, a polyester or polyamide can be produced using an organic acid having two carboxyl groups in the molecule (dicarboxylic acid), such as adipic acid, muconic acid, succinic acid, sebacic acid, itaconic acid, 2,5-furandicarboxylic acid or terephthalic acid. Production of a polyester or polyamide using a dicarboxylic acid as a raw material requires a diol or diamine, respectively, and these may be derived from either a biomass resource or petroleum.

As a method for producing a polyester using a dicarboxylic acid as a raw material, a known method may be used as it is, and, for example, the polyester can be produced by esterification reaction or ester exchange reaction of a dicarboxylic acid or a dicarboxylic acid composed of its ester-forming derivative with a diol, followed by polycondensation reaction. Either a solution reaction using a solvent or a melting reaction by heat melting may be employed, and a melting reaction is preferred in view of efficiently obtaining a high-quality polyester. The catalyst and the solvent used for the reaction may be optimized for the diol and the dicarboxylic acid. Further, for the esterification reaction or ester exchange reaction, and the subsequent polycondensation reaction, either a batch method or continuous method may be employed. In each reaction, the reaction vessel is not limited, and examples of the reaction vessel that may be used include stirring-vessel-type reaction vessels, mixer type reaction vessels, tower type reaction vessels and extruder type reaction vessels. Two or more of these reaction vessels may be used in combination.

In the esterification reaction or ester exchange reaction, and the subsequent polycondensation reaction, a catalyst may be used for promoting the reaction. Preferred specific examples of a compound that may be used as the catalyst include titanium compounds, tin compounds, aluminum compounds, calcium compounds, lithium compounds, magnesium compounds, cobalt compounds, manganese compounds, antimony compounds, germanium compounds and zinc compounds, with which high reaction activity can be achieved and the reaction rate and the yield of the obtained polyester can be increased. Examples of the ester exchange catalyst include alkali metal acetates. Examples of the polymerization catalyst include antimony oxide hardly containing germanium oxide, bismuth or the like; compounds of a transition metal such as cobalt; and alkoxy titanates. In particular, in view of reducing the reaction time to allow efficient production, titanium compounds, tin compounds, aluminum compounds, antimony compounds and germanium compounds are preferred; in view of obtaining a polyester whose crystallization property can be easily controlled and which is excellent in qualities such as thermal stability, hydrolysis resistance and thermal conductivity, titanium compounds and/or tin compounds are more preferred; and in view of decreasing the environmental stress, titanium compounds are still more preferred. Examples of the titanium compounds include titanate esters such as tetra-n-propyl ester, tetra-n-butyl ester, tetraisopropyl ester, tetraisobutyl ester, tetra-tert-butyl ester, cyclohexyl ester, phenyl ester, benzyl ester and tolyl ester, and mixed esters thereof. In particular, tetrapropyl titanate, tetrabutyl titanate and tetraisopropyl titanate are preferred in view of efficient production of polyester resins, and tetra-n-butyl titanate and the like are especially preferably used. Examples of the tin compounds include monobutyltin oxide, dibutyltin oxide, methylphenyltin oxide, tetraethyltin oxide, hexaethylditin oxide, cyclohexahexylditin oxide, didodecyltin oxide, triethyltin hydroxide, triphenyltin hydroxide, triisobutyltin acetate, dibutyltin diacetate, diphenyltin dilaurate, monobutyltin trichloride, dibutyltin dichloride, tributyltin chloride, dibutyltin sulfide and butylhydroxytin oxide, and methylstannoic acid, ethylstannoic acid and butylstannoic acid. Among these, in view of efficient production of polyesters, monoalkyltin compounds are especially preferably used. Each of these compounds as catalysts may be used alone, or two or more of these may be used in combination, in the esterification reaction or ester exchange reaction, and the subsequent polycondensation reaction. In terms of the timing of addition of the compound(s), the compound(s) is/are added by any of a method in which the compound(s) is/are added immediately after addition of the raw material, a method in which the compound(s) is/are added at the same time as the raw material, and a method in which the compound(s) is/are added during the reaction. In cases where the compound to be used as a catalyst is a titanium compound, the amount of the compound added is preferably within the range of 0.01 1 to 0.3 part by weight with respect to 100 parts by weight of the polyester produced, and, in view of the thermal stability, color and reactivity of the polymer, the amount is more preferably within the range of 0.02 to 0.2 part by weight, still more preferably within the range of 0.03 to 0.15 part by weight.

Specific examples of the organic acid polyester include the following polyesters.

Examples of polyesters produced using as a raw material a dicarboxylic acid composition comprising succinic acid as a major component include polyesters with ethylene glycol (polyethylene succinate), polyesters with 1,2-propanediol, polyesters with 1,3-propanediol (polytrimethylene succinate), polyesters with 1,4-butanediol (polybutylene succinate), and polyesters with 2,3-propanediol.

Examples of polyesters produced using as a raw material a dicarboxylic acid composition comprising adipic acid as a major component include polyesters with ethylene glycol (polyethylene adipate), polyesters with 1,2-propanediol, polyesters with 1,3-propanediol (polytrimethylene adipate), polyesters with 1,4-butanediol (polybutylene adipate), and polyesters with 2,3-propanediol.

Examples of polyesters produced using as a raw material a dicarboxylic acid composition comprising sebacic acid as a major component include polyesters with ethylene glycol (polyethylene sebacinate), polyesters with 1,2-propanediol, polyesters with 1,3-propanediol (polytrimethylene sebacinate), polyesters with 1,4-butanediol (polybutylene sebacinate), and polyesters with 2,3-propanediol.

Examples of polyesters produced using as a raw material a dicarboxylic acid composition comprising 2,5-furandicarboxylic acid as a major component include polyesters with ethylene glycol, polyesters with 1,2-propanediol, polyesters with 1,3-propanediol, polyesters with 1,4-butanediol, and polyesters with 2,3-propanediol.

Examples of polyesters produced using as a raw material a dicarboxylic acid composition comprising itaconic acid as a major component include polyesters with ethylene glycol, polyesters with 1,2-propanediol, polyesters with 1,3-propanediol, polyesters with 1,4-butanediol, and polyesters with 2,3-propanediol.

Examples of polyesters produced using as a raw material a dicarboxylic acid composition comprising terephthalic acid as a major component include polyesters with ethylene glycol (polyethylene terephthalate), polyesters with 1,2-propanediol, polyesters with 1,3-propanediol (polytrimethylene terephthalate), polyesters with 1,4-butanediol (polybutylene terephthalate), and polyesters with 2,3-propanediol.

[Organic Acid Polyamide]

As the method for producing an organic acid polyamide using an organic acid obtained by the present invention as a raw material, a known method may be used as it is, and, more specifically, a method in which the above-described dicarboxylic acid and diamine are polycondensed is applied (see Osamu Fukumoto ed., "Polyamide Resin Handbook", Nikkan Kogyo Shimbun, Ltd. (January, 1998) or JP 2004-75932 A).

Specific examples of the organic acid polyamide include the following polyamides.

Examples of polyamides produced using as a raw material a dicarboxylic acid composition comprising succinic acid as a major component include polyamides with hexamethylenediamine (polyhexamethylene succinamide, nylon 64), polyamides with 1,5-pentanediamine (polypentamethylene succinamide, nylon 54), polyamides with 1,4-butanediamine (polytetramethylene succinamide, nylon 44), polyamides with 1,3-propanediamine (polytrimethylene succinamide, nylon 34), polyamides with 1,2-propanediamine, polyamides with 1,2-ethylenediamine (polyethylene succinamide, nylon 24), and polyamides with o-phenylenediamine, m-phenylenediamine or p-phenylenediamine.

Examples of polyamides produced using as a raw material a dicarboxylic acid composition comprising adipic acid as a major component include polyamides with hexamethylenediamine (polyhexamethylene adipamide, nylon 66), polyamides with 1,5-pentanediamine (polypentamethylene adipamide, nylon 56), polyamides with 1,4-butanediamine (polytetramethylene adipamide, nylon 46), polyamides with 1,3-propanediamine (polytrimethylene adipamide, nylon 36), polyamides with 1,2-propanediamine, polyamides with 1,2-ethylenediamine (polyethylene adipamide, nylon 26), and polyamides with o-phenylenediamine, m-phenylenediamine or p-phenylenediamine.

Examples of polyamides produced using as a raw material a dicarboxylic acid composition comprising sebacic acid as a major component include polyamides with hexamethylenediamine (polyhexamethylene sebacimide, nylon 610), polyamides with 1,5-pentanediamine (polypentamethylene sebacimide, nylon 510), polyamides with 1,4-butanediamine (polytetramethylene sebacimide, nylon 410), polyamides with 1,3-propanediamine (polytrimethylene sebacimide, nylon 310), polyamides with 1,2-propanediamine, polyamides with 1,2-ethylenediamine (polyethylene sebacimide, nylon 210), and polyamides with o-phenylenediamine, m-phenylenediamine or p-phenylenediamine.

Examples of polyamides produced using as a raw material a dicarboxylic acid composition comprising 2,5-furandicarboxylic acid as a major component include polyamides with hexamethylenediamine, polyamides with 1,5-pentanediamine, polyamides with 1,4-butanediamine, polyamides with 1,3-propanediamine, polyamides with 1,2-propanediamine, polyamides with 1,2-ethylenediamine, and polyamides with o-phenylenediamine, m-phenylenediamine or p-phenylenediamine.

Examples of polyamides produced using as a raw material a dicarboxylic acid composition comprising itaconic acid as a major component include polyamides with hexamethylenediamine, polyamides with 1,5-pentanediamine, polyamides with 1,4-butanediamine, polyamides with 1,3-propanediamine, polyamides with 1,2-propanediamine, polyamides with 1,2-ethylenediamine, and polyamides with o-phenylenediamine, m-phenylenediamine or p-phenylenediamine.

Examples of polyamides produced using as a raw material a dicarboxylic acid composition comprising terephthalic acid as a major component include polyamides with hexamethylenediamine (polyhexamethylene terephthalamide, nylon 6T), polyamides with 1,5-pentanediamine (polypentamethylene terephthalamide, nylon 5T), polyamides with 1,4-butanediamine (polytetramethylene terephthalamide, nylon 4T), polyamides with 1,3-propanediamine (polytrimethylene terephthalamide, nylon 3T), polyamides with 1,2-propanediamine, polyamides with 1,2-ethylenediamine (polyethylene terephthalamide, nylon 2T), and polyamides with o-phenylenediamine, m-phenylenediamine or p-phenylenediamine.

The organic acid polymer obtained by the present invention is more excellent in the color, weight average molecular weight, melting point, and weight reduction rate upon heating than biomass resource-derived organic acid polymers obtained by conventional methods. In particular, in cases where a polymer is used for a fiber, film or molded product, the polymer preferably has an APHA value of not more than 15 in terms of the color. By the present invention, a biomass resource-derived organic acid polymer that is excellent in color and has an APHA value of not more than 15 can be obtained.

EXAMPLES

The present invention is described below by way of Examples, but the present invention is not limited to the Examples below.

(Reference Example 1) Yeast Strain Having L-Lactic Acid Fermentation Capacity

HI003, which is an L-lactic acid fermentation yeast strain described in Reference Example 1 of WO2009/099044, was used as a microorganism for production of L-lactic acid.

(Reference Example 2) Production of L-Lactic Acid by Batch Fermentation

Using the HI003 strain of Reference Example 1 and a raw sugar medium (70 g/L Yutosei (manufactured by MUSO Co., Ltd.), 1.5 g/L ammonium sulfate), a batch fermentation test was carried out under the following culture conditions by the method described below. The medium was autoclaved (121°C, 15 minutes) before use.

[Culture Conditions]

Reaction vessel capacity (volume of the lactic acid fermentation medium): 30 (L); temperature control: 32 (°C); aeration rate in the reaction vessel: 0.1 (L/min.); reaction vessel stirring rate: 200 (rpm); pH control: adjustment to pH 6.5 with 1 N calcium hydroxide.

[Culture Method]

The HI003 strain was cultured in 5 ml of the raw sugar medium in a test tube overnight with shaking (pre-preculture). The obtained pre-preculture liquid was inoculated to 100 ml of a fresh portion of the raw sugar medium, and cultured in a 500-ml Sakaguchi flask for 24 hours with shaking (preculture). The temperature control and the pH control were carried out, and fermentation culture was performed.
The concentration and the optical purity of the lactic acid obtained by the batch fermentation in Reference Example 2 were evaluated under the following measurement conditions by HPLC.

[Measurement of Lactic Acid Concentration]

Column: Shim-Pack SPR-H (manufactured by Shimadzu Corporation)
Mobile phase: 5 mM p-Toluenesulfonic acid (flow rate, 0.8 mL/min.)
Reaction solution: 5 mM p-Toluenesulfonic acid, 20 mM bis-Tris, 0.1 mM EDTA 2Na (flow rate, 0.8 mL/min.)
Detection method: Electric conductivity
Temperature: 45°C

[Optical Purity of L-Lactic Acid]

The L-lactic acid and D-lactic acid concentrations were measured under the following conditions.

Column: TSK-gel Enantio L1 (manufactured by Tosoh Corporation)
Mobile phase: 1 mM Aqueous copper sulfate solution
Flow rate: 1.0 ml/min.
Detection method: UV 254 nm
Temperature: 30°C

Subsequently, the optical purity was calculated according to the following equation.

$$\text{Optical purity (\% e.e.)} = 100 \times (L\text{-}D) \text{ or } (D\text{-}L) / (L\text{+}D)$$

In the equation, L represents the concentration of L-lactic acid, and D represents the concentration of D-lactic acid. An optical purity of 100% (100% ee) herein means that no peak for the enantiomer could be detected in the HPLC for measuring the optical purity described later.

As a result of the batch fermentation for 50 hours, the concentration of lactic acid accumulated was 45 to 49 g/L, and the optical purity was 100% for L-lactic acid.

(Reference Example 3) Providing Test Solution (Raw Aqueous Lactic Acid Solution) from Yeast L-Lactic Acid Fermentation Culture Liquid

Yeast cells were removed from 30 L of the L-lactic acid culture liquid prepared in Reference Example 2 using a centrifuge, and 95% sulfuric acid (manufactured by Sigma Aldrich) was added to the obtained supernatant to pH 2.5, followed by stirring the resulting mixture for 2 hours. The produced calcium sulfate was removed by suction filtration, and the obtained filtrate was passed through a column packed with a strong anion-exchange resin ("DIAION SA10A", manufactured by Mitsubishi Chemical Corporation) in the downflow direction. The resultant was then passed through a column packed with a strong cation-exchange resin ("DIAION SK1B" manufactured by Mitsubishi Chemical Corporation) in the downflow direction. Subsequently, the resultant was filtered through a nanofiltration membrane (4-inch spiral element "SU-610", manufactured by Toray Industries, Inc.), to obtain 28 L of a raw aqueous lactic acid solution. Subsequently, the solution was concentrated to 47 wt% using a thin-film evaporator (manufactured by Tokyo Rikakikai Co., Ltd.), to provide a test solution. The color intensity of the test solution was analyzed according to JIS K 0071-1 using a colorimeter (manufactured by Nippon Denshoku Industries Co., Ltd.) in terms of the APHA unit color number. As a result, the AHPA value of the test solution was found to be 150. This test solution was subjected to distillation at 130°C under a reduced pressure of 133 Pa. The APHA value and the optical purity of the lactic acid obtained by the distillation are shown in Table 1.

(Examples 1 to 68) Experiment for Addition of Oxidizing Agent to Raw Aqueous Lactic Acid Solution, Experiment for Distillation under Reduced Pressure, and Polylactic Acid Polymerization Experiment

In a glass Schlenk flask, 500 mL of the test solution obtained in Reference Example 3 was placed. Each of various oxidizing agents (Examples 1 to 16, 5% aqueous sodium hypochlorite solution; Examples 17 to 36, 30% hydrogen peroxide solution; Examples 37 to 52, sodium chlorite; Examples 53 to 68, tert-butylhydroperoxide; all reagents were manufactured by Wako Pure Chemical Industries, Ltd.) was added to the test solution, and the resulting mixture was stirred at 25°C (without heating), 60°C, 100°C or 180°C for 2 hours. The types and the concentrations of the oxidizing agents added, and the heating conditions; and the results of measurement, according to the measurement methods described in the above Reference Examples, of the APHA values and the optical purities of the lactic acids after oxidizing agent treatment, and the APHA values and the optical purities of the lactic acids obtained after distillation at 130°C under a reduced pressure of 133 Pa; are shown in Tables 1 and 2. In cases where the oxidizing agent was in the form of an aqueous solution, the concentration of the oxidizing agent added to the test solution was measured in terms of the pure content excluding water. The concentration of the oxidizing agent added was calculated according to Equation 1.

$$\text{Concentration of oxidizing agent added (\%)} = (\text{weight of oxidizing agent excluding water} / \text{weight of lactic acid excluding water}) \times 100 \ldots \text{(Equation 1)}$$

[Table 1]

| | Oxidizing agent | Concentration of the agent added (%) | Heating temperature (°C) | Before distillation | | After distillation | |
|---|---|---|---|---|---|---|---|
| | | | | APHA | Optical purity (‰e.e.) | APHA | Optical purity (‰e.e.) |
| Reference Example 3 | No addition | 0 | No heating | 150 | 100 | 31 | 100 |
| Example 1 | 5% Aqueous sodium hypochlorite solution | 1 | No heating | 28 | 100 | 4 | 100 |
| Example 2 | | 1 | 60 | 21 | 100 | 3 | 100 |
| Example 3 | | 1 | 100 | 17 | 100 | 3 | 100 |
| Example 4 | | 1 | 180 | 18 | 100 | 3 | 100 |
| Example 5 | | 0.5 | No heating | 46 | 100 | 5 | 100 |
| Example 6 | | 0.5 | 60 | 34 | 100 | 3 | 100 |
| Example 7 | | 0.5 | 100 | 28 | 100 | 3 | 100 |
| Example 8 | | 0.5 | 180 | 25 | 100 | 3 | 100 |
| Example 9 | | 0.1 | No heating | 73 | 100 | 5 | 100 |
| Example 10 | | 0.1 | 60 | 65 | 100 | 4 | 100 |
| Example 11 | | 0.1 | 100 | 61 | 100 | 4 | 100 |
| Example 12 | | 0.1 | 180 | 59 | 100 | 5 | 100 |
| Example 13 | | 0.01 | No heating | 124 | 100 | 12 | 100 |
| Example 14 | | 0.01 | 60 | 115 | 100 | 11 | 100 |
| Example 15 | | 0.01 | 100 | 101 | 100 | 11 | 100 |
| Example 16 | | 0.01 | 180 | 97 | 100 | 9 | 100 |
| Example 17 | 30% Hydrogen peroxide solution | 5 | No heating | 94 | 100 | 7 | 100 |
| Example 18 | | 5 | 60 | 36 | 100 | 6 | 100 |
| Example 19 | | 5 | 100 | 18 | 100 | 3 | 100 |
| Example 20 | | 5 | 180 | 17 | 100 | 3 | 100 |
| Example 21 | | 1 | No heating | 134 | 100 | 8 | 100 |
| Example 22 | | 1 | 60 | 65 | 100 | 3 | 100 |
| Example 23 | | 1 | 100 | 36 | 100 | 3 | 100 |
| Example 24 | | 1 | 180 | 35 | 100 | 2 | 100 |
| Example 25 | | 0.5 | No heating | 148 | 100 | 6 | 100 |
| Example 26 | | 0.5 | 60 | 73 | 100 | 4 | 100 |
| Example 27 | | 0.5 | 100 | 52 | 100 | 3 | 100 |
| Example 28 | | 0.5 | 180 | 55 | 100 | 3 | 100 |
| Example 29 | | 0.1 | No heating | 146 | 100 | 7 | 100 |
| Example 30 | | 0.1 | 60 | 135 | 100 | 6 | 100 |
| Example 31 | | 0.1 | 100 | 132 | 100 | 4 | 100 |
| Example 32 | | 0.1 | 180 | 183 | 100 | 4 | 100 |
| Example 33 | | 0.01 | No heating | 151 | 100 | 8 | 100 |
| Example 34 | | 0.01 | 60 | 157 | 100 | 8 | 100 |
| Example 35 | | 0.01 | 100 | 183 | 100 | 5 | 100 |
| Example 36 | | 0.01 | 180 | 199 | 100 | 6 | 100 |

[Table 2]

| | Oxidizing agent | Concentration of the agent added (%) | Heating temperature (°C) | Before distillation | | After distillation | |
|---|---|---|---|---|---|---|---|
| | | | | APHA | Optical purity (%e.e.) | APHA | Optical purity (%e.e.) |
| Example 37 | Sodium chlorite | 1 | No heating | 103 | 100 | 5 | 100 |
| Example 38 | | 1 | 60 | 52 | 100 | 3 | 100 |
| Example 39 | | 1 | 100 | 26 | 100 | 3 | 100 |
| Example 40 | | 1 | 180 | 24 | 100 | 3 | 100 |
| Example 41 | | 0.5 | No heating | 120 | 100 | 3 | 100 |
| Example 42 | | 0.5 | 60 | 73 | 100 | 4 | 100 |
| Example 43 | | 0.5 | 100 | 49 | 100 | 3 | 100 |
| Example 44 | | 0.5 | 180 | 32 | 100 | 3 | 100 |
| Example 45 | | 0.1 | No heating | 123 | 100 | 7 | 100 |
| Example 46 | | 0.1 | 60 | 99 | 100 | 5 | 100 |
| Example 47 | | 0.1 | 100 | 82 | 100 | 4 | 100 |
| Example 48 | | 0.1 | 180 | 70 | 100 | 4 | 100 |
| Example 49 | | 0.01 | No heating | 138 | 100 | 11 | 100 |
| Example 50 | | 0.01 | 60 | 135 | 100 | 8 | 100 |
| Example 51 | | 0.01 | 100 | 124 | 100 | 8 | 100 |
| Example 52 | | 0.01 | 180 | 112 | 100 | 7 | 100 |
| Example 53 | tert-Butylhydroperoxide | 1 | No heating | 136 | 100 | 19 | 100 |
| Example 54 | | 1 | 60 | 129 | 100 | 18 | 100 |
| Example 55 | | 1 | 100 | 153 | 100 | 10 | 100 |
| Example 56 | | 1 | 180 | 167 | 100 | 9 | 100 |
| Example 57 | | 0.5 | No heating | 141 | 100 | 23 | 100 |
| Example 58 | | 0.5 | 60 | 139 | 100 | 20 | 100 |
| Example 59 | | 0.5 | 100 | 172 | 100 | 16 | 100 |
| Example 60 | | 0.5 | 180 | 191 | 100 | 10 | 100 |
| Example 61 | | 0.1 | No heating | 143 | 100 | 25 | 100 |
| Example 62 | | 0.1 | 60 | 166 | 100 | 21 | 100 |
| Example 63 | | 0.1 | 100 | 184 | 100 | 20 | 100 |
| Example 64 | | 0.1 | 180 | 208 | 100 | 15 | 100 |
| Example 65 | | 0.01 | No heating | 151 | 100 | 28 | 100 |
| Example 66 | | 0.01 | 60 | 195 | 100 | 27 | 100 |
| Example 67 | | 0.01 | 100 | 201 | 100 | 21 | 100 |
| Example 68 | | 0.01 | 180 | 210 | 100 | 19 | 100 |

Thereafter, lactic acids whose APHA was not more than 10 after the distillation were subjected to a polylactic acid polymerization test as described below.

In a reaction vessel equipped with a stirrer, 150 g of lactic acid was heated at 800 Pa at 160°C for 3.5 hours, to obtain oligomers. Subsequently, 0.12 g oftin(II) acetate (manufactured by Kanto Chemical Co., Inc.) and 0.33 g of methanesulfonic acid (manufactured by Wako Pure Chemical Industries, Ltd.) were added to the oligomers, and the resulting mixture was heated at 500 Pa at 180°C for 7 hours, to obtain a prepolymer. Subsequently, the prepolymer was heated in an oven at 120 °C for 2 hours to allow crystallization. The obtained prepolymer was pulverized using a hammer crusher, and sieved to obtain a powder with an average particle size of 0.1 mm. In the solid phase polymerization step, 50 g of the prepolymer was placed in an oven connected to an oil rotary pump, and heat treatment was performed under reduced pressure. In this treatment, the pressure was 50 Pa, the heating temperatures were: 140°C for 10 hours, 150°C for 10 hours, and 160°C for 20 hours. The obtained polylactic acid was subjected to analysis of the weight average molecular weight by GPC, analysis of the melting point by DSC, analysis of the weight reduction rate upon heating by TG, and measurement of the degree of coloration. The results are shown in Tables 3 and 4.

[Analysis of Weight Average Molecular Weight of Polylactic Acid]

The weight average molecular weight (Mw) of the polylactic acid is the value of the weight average molecular weight measured by gel permeation chromatography (GPC) in terms of a standard polymethyl methacrylate. In the GPC measurement, HLC 8320GPC (manufactured by Tosoh Corporation) was used as the GPC system, and two linearly connected TSK-GEL Super HM-M columns (manufactured by Tosoh Corporation) were used. Detection was carried out with a differential refractometer. In terms of the measurement conditions, the flow rate was 0.35 mL/min.; hexafluoroisopropanol was used as the solvent; and 0.02 mL of a sample solution at a concentration of 1 mg/mL was injected.

[Analysis of Melting Point of Polylactic Acid]

The melting point of the polylactic acid was measured using a differential scanning calorimeter DSC7020 (manufactured by SII Nanotechnology Inc.). The measurement was carried out with 10 mg of the sample under nitrogen atmosphere at a rate of temperature increase of 20°C/min.

[Analysis of Weight Reduction Rate upon Heating of Polylactic Acid]

The weight reduction rate upon heating of the polylactic acid was measured using a thermogravimetry/differential thermal analyzer TG/DTA7200 (manufactured by SII Nanotechnology Inc.). The measurement was carried out with 10 mg the sample under nitrogen atmosphere at a constant temperature of 200°C for a heating time of 30 minutes.

[Measurement of Degree of Coloration of Polylactic Acid]

For measuring the degree of coloration of the polylactic acid produced by polymerization, 0.4 g of the polylactic acid was dissolved in 25 mL of chloroform and analyzed according to JIS K 0071-1 using a colorimeter (manufactured by Nippon Denshoku Industries Co., Ltd.) in terms of the APHA unit color number.

[Table 3]

| | Lactic acid | | Polylactic acid | | | |
|---|---|---|---|---|---|---|
| | Oxidizing agent | After distillation APHA | Weight average molecular weight (×1000) | Melting point (°C) | Weight reduction rate upon heating (%) | APHA |
| Reference Example 3 | No addition | 31 | 158 | 160.9 | 35 | 28 |
| Example 1 | 5% Aqueous sodium hypochlorite solution | 4 | 176 | 163.8 | 25 | 5 |
| Example 2 | | 3 | 188 | 164.4 | 26 | 4 |
| Example 3 | | 3 | 205 | 164.6 | 24 | 3 |
| Example 4 | | 3 | 201 | 164.8 | 24 | 4 |
| Example 5 | | 5 | 181 | 164.2 | 25 | 5 |
| Example 6 | | 3 | 192 | 163.9 | 26 | 5 |
| Example 7 | | 3 | 191 | 164.6 | 25 | 6 |
| Example 8 | | 3 | 215 | 164.6 | 28 | 5 |
| Example 9 | | 5 | 166 | 163.0 | 27 | 6 |
| Example 10 | | 4 | 177 | 163.4 | 29 | 8 |
| Example 11 | | 4 | 194 | 163.5 | 27 | 7 |
| Example 12 | | 5 | 178 | 163.6 | 30 | 8 |
| Example 16 | | 9 | 169 | 163.3 | 31 | 4 |
| Example 17 | 30% Hydrogen peroxide solution | 7 | 202 | 165.9 | 24 | 3 |
| Example 18 | | 6 | 198 | 165.8 | 25 | 3 |
| Example 19 | | 3 | 213 | 166.0 | 23 | 3 |
| Example 20 | | 3 | 237 | 166.2 | 21 | 3 |
| Example 21 | | 4 | 208 | 166.1 | 23 | 3 |
| Example 22 | | 3 | 210 | 166.2 | 24 | 3 |
| Example 23 | | 3 | 226 | 166.4 | 21 | 3 |
| Example 24 | | 2 | 241 | 166.5 | 21 | 3 |
| Example 25 | | 6 | 217 | 166.0 | 23 | 3 |
| Example 26 | | 4 | 231 | 166.2 | 25 | 3 |
| Example 27 | | 3 | 223 | 166.3 | 26 | 3 |
| Example 28 | | 3 | 235 | 166.1 | 28 | 4 |
| Example 29 | | 7 | 224 | 165.6 | 27 | 3 |
| Example 30 | | 6 | 210 | 166.0 | 26 | 3 |
| Example 31 | | 4 | 235 | 165.9 | 29 | 4 |
| Example 32 | | 4 | 234 | 165.7 | 31 | 4 |
| Example 33 | | 8 | 218 | 165.5 | 29 | 5 |
| Example 34 | | 8 | 222 | 165.9 | 28 | 5 |
| Example 35 | | 5 | 226 | 165.7 | 27 | 4 |
| Example 36 | | 6 | 222 | 165.8 | 26 | 4 |

[Table 4]

| | Lactic acid | | | Polylactic acid | | | |
|---|---|---|---|---|---|---|---|
| | Oxidizing agent | After distillation APHA | Weight average molecular weight (×1000) | Melting point (°C) | Weight reduction rate upon heating (%) | APHA |
| Example 37 | Sodium chlorite | 5 | 189 | 163.6 | 27 | 6 |
| Example 38 | | 3 | 201 | 163.7 | 28 | 6 |
| Example 39 | | 3 | 195 | 164.1 | 30 | 7 |
| Example 40 | | 3 | 198 | 164.2 | 28 | 5 |
| Example 41 | | 3 | 196 | 163.8 | 29 | 6 |
| Example 42 | | 4 | 203 | 163.6 | 27 | 5 |
| Example 43 | | 3 | 187 | 163.9 | 32 | 5 |
| Example 44 | | 3 | 190 | 164.0 | 28 | 5 |
| Example 45 | | 7 | 184 | 163.4 | 27 | 7 |
| Example 46 | | 5 | 182 | 163.4 | 31 | 5 |
| Example 47 | | 4 | 179 | 164.0 | 30 | 5 |
| Example 48 | | 4 | 191 | 163.7 | 28 | 6 |
| Example 50 | | 8 | 183 | 163.7 | 30 | 10 |
| Example 51 | | 8 | 178 | 163.4 | 31 | 7 |
| Example 52 | | 7 | 185 | 163.5 | 28 | 8 |
| Example 55 | tert-Butylhydroperoxide | 10 | 184 | 162.5 | 31 | 14 |
| Example 56 | | 9 | 175 | 162.9 | 29 | 11 |
| Example 60 | | 10 | 176 | 162.8 | 34 | 13 |

(Comparative Examples 1 to 3) Heating Experiment of Raw Aqueous Lactic Acid Solution, and Experiment for Distillation under Reduced Pressure

In a glass Schlenk flask, 500 mL of the test solution obtained in Reference Example 3 was placed. Without addition of an oxidizing agent, the solution was stirred at 60°C, 100°C or 180°C for 2 hours. The APHA value and the optical purity of the lactic acid after heating, and the APHA value and the optical purity of the lactic acid obtained after distillation at 130°C under a reduced pressure of 133 Pa are shown in Table 5. Subsequently, 150 g of each lactic acid obtained by the distillation was subjected to a polymerization test in the same manner as in Examples 1 to 68. The results are shown in Table 6.

(Comparative Examples 4 to 19) Experiment for Addition of Reducing Agent to Raw Aqueous Lactic Acid Solution, Experiment for Distillation under Reduced Pressure, and Polylactic Acid Polymerization Experiment

In a glass Schlenk flask, 500 mL of the test solution obtained in Reference Example 3 was placed. Each of various reducing agents was added to the test solution, and the resulting mixture was stirred at 25°C for 2 hours. The types of the reducing agents added; and the APHA values and the optical purities of the lactic acids after reducing agent treatment, and the APHA values and the optical purities of the lactic acids obtained after distillation at 130°C under a reduced pressure of 133 Pa; are shown in Table 5. Further, a polymerization test was carried out in the same manner as in Examples 1 to 68 for 150 g of each of the lactic acids obtained by distillation in Comparative Example 4, Comparative Example 8, Comparative Example 12 and Comparative Example 16. The results are shown in Table 6.

(Comparative Example 20) Hydrogen Reduction Treatment of Raw Lactic Acid Solution and Experiment for Distillation under Reduced Pressure, and Polylactic Acid Polymerization Experiment

In a glass Schlenk flask, 200 mL of the test solution obtained in Reference Example 3 was placed. As described in JP 3880175 B, 5% palladium-alumina (manufactured by NE Chemcat Corporation) as a catalyst was added to the test solution, and the atmosphere in the reaction container was replaced with nitrogen. Subsequently, the atmosphere in the reaction container was replaced with 200 mL of hydrogen, and catalytic reduction treatment was performed at normal pressure with vigorous stirring. The reduction treatment was finished upon completion of consumption of the hydrogen, and the catalyst was then removed by filtration using a qualitative filter paper NO2 (ADVANTEC). Although the obtained solution had a reduced degree of coloration with an APHA of 138, the solution had a reduced optical purity of 98.8% e.e. By distillation of the filtrate in the same manner as in Examples 1 to 68, the APHA value became 25, and the optical purity became 99.0% e.e. A polymerization test was carried out in the same manner as in Examples 1 to 68 with 150 g of the lactic acid obtained by distillation. The results are shown in Table 6.

[Table 5]

| | Reducing agent | Heating temperature (°C) | Concentration of the agent added (%) | Before distillation | | After distillation | |
|---|---|---|---|---|---|---|---|
| | | | | APHA | Optical purity (%e.e.) | APHA | Optical purity (%e.e.) |
| Reference Example 3 | No addition | No heating | 0 | 150 | 100 | 31 | 100 |
| Comparative Example 1 | No addition | 60 | 0 | 179 | 100 | 29 | 100 |
| Comparative Example 2 | | 100 | 0 | 204 | 100 | 33 | 100 |
| Comparative Example 3 | | 180 | 0 | 225 | 100 | 35 | 100 |
| Comparative Example 4 | NaBH$_4$ | No heating | 1 | 206 | 98.3 | 28 | 98.8 |
| Comparative Example 5 | | | 0.5 | 214 | 98.7 | 29 | 98.8 |
| Comparative Example 6 | | | 0.1 | 220 | 99.1 | 33 | 99.1 |
| Comparative Example 7 | | | 0.01 | 228 | 99.4 | 35 | 99.5 |
| Comparative Example 8 | NaBH (OAc)$_3$ | No heating | 1 | 213 | 99.2 | 27 | 99.2 |
| Comparative Example 9 | | | 0.5 | 214 | 99.5 | 30 | 99.5 |
| Comparative Example 10 | | | 0.1 | 218 | 99.8 | 26 | 99.8 |
| Comparative Example 11 | | | 0.01 | 222 | 99.9 | 29 | 99.9 |
| Comparative Example 12 | BH$_3$· NMe$_3$ | No heating | 1 | 145 | 99.4 | 15 | 99.3 |
| Comparative Example 13 | | | 0.5 | 183 | 99.7 | 27 | 99.7 |
| Comparative Example 14 | | | 0.1 | 203 | 99.9 | 24 | 99.9 |
| Comparative Example 15 | | | 0.01 | 221 | 99.9 | 31 | 99.9 |

(continued)

|  | Reducing agent | Heating temperature (°C) | Concentration of the agent added (%) | Before distillation | | After distillation | |
|---|---|---|---|---|---|---|---|
|  |  |  |  | APHA | Optical purity (%e.e.) | APHA | Optical purity (%e.e.) |
| Comparative Example 16 | Hydrazine monohydrate | No heating | 1 | >500 | 99.9 | 36 | 99.9 |
| Comparative Example 17 |  |  | 0.5 | 392 | 99.9 | 30 | 99.9 |
| Comparative Example 18 |  |  | 0.1 | 297 | 99.9 | 33 | 99.9 |
| Comparative Example 19 |  |  | 0.01 | 249 | 99.9 | 29 | 99.9 |
| Comparative Example 20 | Hydrogen | No heating |  | 198 | 98.8 | 25 | 99 |

[Table 6]

| | Lactic acid used | Polylactic acid | | | |
|---|---|---|---|---|---|
|  | After distillation APHA | Weight average molecular weight (×1000) | Melting point (°C) | Weight reduction rate upon heating(%) | APHA |
| Reference Example 3 | 31 | 158 | 160.9 | 35 | 28 |
| Comparative Example 4 | 28 | 153 | 160.3 | 38 | 35 |
| Comparative Example 8 | 27 | 162 | 161.2 | 36 | 34 |
| Comparative Example 12 | 15 | 168 | 162.1 | 26 | 27 |
| Comparative Example 16 | 36 | 142 | 160.2 | 37 | 43 |
| Comparative Example 20 | 25 | 171 | 161.7 | 32 | 32 |

From the results of the above Examples and Comparative Examples, it became clear that addition of an oxidizing agent to a raw lactic acid solution containing a colored component allows removal of the colored impurity and improvement of properties of polylactic acid produced by polymerization, without decreasing the optical purity of the lactic acid.

(Reference Example 4) Preparation of Succinic Acid Culture Liquid

In an anaerobic glove box, 1 mL of 30 mM sodium carbonate and 0.15 mL of 180 mM sulfuric acid were added to 100 mL of the medium for seed culture composed of 20 g/L glucose, 10 g/L polypeptone, 5 g/L yeast extract, 3 g/L dipotassium hydrogen phosphate, 1 g/L sodium chloride, 1 g/L ammonium sulfate, 0.2 g/L magnesium chloride hexahydrate and 0.2 g/L calcium chloride dihydrate that was heat-sterilized at 121 °C at 2 atm for 20 minutes, and 0.5 mL of the reducing solution composed of 0.25 g/L cysteine-HCl and 0.25 g/L sodium sulfide was further added to the resulting mixture. *Anaerobiospirillum succiniciproducens* ATCC53488 was inoculated to the prepared medium, and static culture was carried out at 39°C overnight to prepare a preculture liquid.

Thereafter, $CO_2$ gas was flown from a sparger at a rate of 10 mL/min. into 3L of the fermentation medium composed of 50 g/L glucose, 10 g/L polypeptone, 5 g/L yeast extract, 1 g/L dipotassium hydrogen phosphate, 0.4 g/L ammonium chloride, 0.2 g/L calcium chloride dihydrate, 0.2 g/L magnesium chloride hexahydrate and 0.001 g/L iron sulfate heptahydrate that was heat-sterilized at 121°C at 2 atm for 20 minutes, and 30 mL of 3M sodium carbonate was then added to the medium, followed by adjusting the pH of the resulting medium to 6.8 with a sulfuric acid solution. Thereafter, 1.5 mL of the reducing solution composed of 0.25 g/L cysteine-HCl and 0.25 g/L sodium sulfide was added to the resulting medium, and 50 mL of the preculture liquid described above was inoculated thereto, followed by performing main culture at a stirring rate of 200 rpm at 39°C for 39 hours. During the culture, the pH of the culture liquid was adjusted to 6.4 using 5 M calcium hydroxide.

As a result of HPLC analysis of 100 L of the succinic acid culture liquid under the following measurement conditions, the amount of succinic acid accumulated was 1150 g.

[HPLC Analysis Conditions]

Column: Shim-Pack SPR-H (manufactured by Shimadzu Corporation), 45°C
Mobile phase: 5 mM p-Toluenesulfonic acid, 0.8 mL/min.
Reaction solution: 5 mM p-Toluenesulfonic acid, 20 mM bis-Tris, 0.1 mM EDTA-2Na (0.8 mL/min.)
Detector: Electric conductivity

(Reference Example 5) Providing Test Solution (Raw Aqueous Succinic Acid Solution)

A culture liquid containing calcium succinate was obtained by heat-sterilizing 100 L of the culture liquid prepared in Reference Example 4 at 120°C for 20 minutes, centrifuging the resultant at 5000×G for 20 minutes and collecting the resulting supernatant. Ultrapure water and 95% sulfuric acid (manufactured by Sigma Aldrich) were added to the culture supernatant until the pH became 2.5, and the produced calcium sulfate was removed by suction filtration to obtain an aqueous succinic acid solution. This was further followed by purification with a strong cation-exchange resin and a strong anion-exchange resin in the same manner as in Reference Example 3. Ultrapure water was added to the purified solution to prepare 1 wt% aqueous succinic acid solution, which was used as a test solution. The color intensity of the test solution was analyzed according to JIS K 0071-1 using a colorimeter (manufactured by Nippon Denshoku industries Co., Ltd.) in terms of the APHA unit color number. As a result, the AHPA value of the test solution was 121. Using a rotary evaporator (manufactured by Tokyo Rikakikai Co., Ltd.), 10 L of the test solution was concentrated at 70°C at 10 kPa, to obtain 1 L of 10 wt% aqueous succinic acid solution. Thereafter, the obtained solution was stirred at 4°C for 12 hours, and precipitated succinic acid crystals were collected by solid-liquid separation by suction filtration. To 1600 g of ultrapure water, 205 g of the wet crystals (water content, 58%) of succinic acid obtained by crystallization were added, and the crystals were dissolved in the ultrapure water, to provide 5 wt% aqueous succinic acid solution. The APHA value of the solution was 29.

(Examples 69 to 136) Experiment for Addition of Oxidizing Agent to Raw Aqueous Succinic Acid Solution, and Crystallization Experiment

In a glass flask, 10 L of the test solution obtained in Reference Example 5 was placed. Each of various oxidizing agents was added to the test solution, and the resulting mixture was stirred at 25°C (without heating), 60°C, 100°C or 180°C for 2 hours. The types and the concentrations of the oxidizing agents added, and the heating conditions; the APHA values after the oxidizing agent treatment; and the APHA values of 5 wt% aqueous succinic acid solutions obtained by concentration, crystallization and redissolution in the same manner as in Reference Example 4; are shown in Tables 7 and 8. In cases where the oxidizing agent was in the form of an aqueous solution, the concentration of the oxidizing agent added to the test solution was measured in terms of the pure content excluding water. The concentration of the oxidizing agent added was calculated according to the calculation equation shown in Equation 2.

$$\text{Concentration of oxidizing agent added (\%)} = (\text{weight of oxidizing agent excluding}$$

$$\text{water / weight of succinic acid excluding water}) \times 100 \ldots \text{(Equation 2)}$$

[Table 7]

| | Oxidizing agent | Concentration of the agent added (%) | Heating temperature (°C) | Before crystallization | After crystallization |
|---|---|---|---|---|---|
| | | | | APHA | |
| Reference Example 5 | No addition | 0 | No heating | 121 | 29 |
| Example 69 | 5% Aqueous sodium hypochlorite solution | 1 | No heating | 31 | 5 |
| Example 70 | | 1 | 60 | 24 | 5 |
| Example 71 | | 1 | 100 | 21 | 4 |
| Example 72 | | 1 | 180 | 18 | 4 |
| Example 73 | | 0.5 | No heating | 42 | 8 |
| Example 74 | | 0.5 | 60 | 36 | 6 |
| Example 75 | | 0.5 | 100 | 32 | 6 |
| Example 76 | | 0.5 | 180 | 25 | 5 |
| Example 77 | | 0.1 | No heating | 61 | 9 |
| Example 78 | | 0.1 | 60 | 57 | 8 |
| Example 79 | | 0.1 | 100 | 44 | 8 |
| Example 80 | | 0.1 | 180 | 39 | 7 |
| Example 81 | | 0.01 | No heating | 87 | 12 |
| Example 82 | | 0.01 | 60 | 84 | 11 |
| Example 83 | | 0.01 | 100 | 78 | 11 |
| Example 84 | | 0.01 | 180 | 69 | 8 |
| Example 85 | 30% Hydrogen peroxide solution | 5 | No heating | 34 | 6 |
| Example 86 | | 5 | 60 | 25 | 6 |
| Example 87 | | 5 | 100 | 17 | 3 |
| Example 88 | | 5 | 180 | 17 | 3 |
| Example 89 | | 1 | No heating | 38 | 6 |
| Example 90 | | 1 | 60 | 32 | 5 |
| Example 91 | | 1 | 100 | 23 | 3 |
| Example 92 | | 1 | 180 | 19 | 3 |
| Example 93 | | 0.5 | No heating | 44 | 5 |
| Example 94 | | 0.5 | 60 | 30 | 4 |
| Example 95 | | 0.5 | 100 | 29 | 4 |
| Example 96 | | 0.5 | 180 | 22 | 3 |
| Example 97 | | 0.1 | No heating | 63 | 8 |
| Example 98 | | 0.1 | 60 | 58 | 6 |
| Example 99 | | 0.1 | 100 | 43 | 6 |
| Example 100 | | 0.1 | 180 | 28 | 4 |
| Example 101 | | 0.01 | No heating | 88 | 10 |
| Example 102 | | 0.01 | 60 | 67 | 7 |
| Example 103 | | 0.01 | 100 | 59 | 6 |
| Example 104 | | 0.01 | 180 | 47 | 5 |

[Table 8]

| | Oxidizing agent | Concentration of the agent added (%) | Heating temperature (°C) | Before crystallization | After crystallization |
|---|---|---|---|---|---|
| | | | | APHA | |
| Example 105 | Sodium chlorite | 1 | No heating | 35 | 10 |
| Example 106 | | 1 | 60 | 34 | 8 |
| Example 107 | | 1 | 100 | 23 | 6 |
| Example 108 | | 1 | 180 | 21 | 6 |
| Example 109 | | 0.5 | No heating | 50 | 14 |
| Example 110 | | 0.5 | 60 | 38 | 10 |
| Example 111 | | 0.5 | 100 | 37 | 9 |
| Example 112 | | 0.5 | 180 | 33 | 7 |
| Example 113 | | 0.1 | No heating | 73 | 18 |
| Example 114 | | 0.1 | 60 | 56 | 16 |
| Example 115 | | 0.1 | 100 | 47 | 13 |
| Example 116 | | 0.1 | 180 | 43 | 12 |
| Example 117 | | 0.01 | No heating | 109 | 24 |
| Example 118 | | 0.01 | 60 | 97 | 21 |
| Example 119 | | 0.01 | 100 | 94 | 19 |
| Example 120 | | 0.01 | 180 | 108 | 20 |
| Example 121 | tert-Butylhydroperoxide | 1 | No heating | 108 | 16 |
| Example 122 | | 1 | 60 | 101 | 12 |
| Example 123 | | 1 | 100 | 76 | 11 |
| Example 124 | | 1 | 180 | 92 | 10 |
| Example 125 | | 0.5 | No heating | 79 | 10 |
| Example 126 | | 0.5 | 60 | 86 | 11 |
| Example 127 | | 0.5 | 100 | 137 | 18 |
| Example 128 | | 0.5 | 180 | 119 | 13 |
| Example 129 | | 0.1 | No heating | 96 | 14 |
| Example 130 | | 0.1 | 60 | 112 | 15 |
| Example 131 | | 0.1 | 100 | 138 | 27 |
| Example 132 | | 0.1 | 180 | 146 | 25 |
| Example 133 | | 0.01 | No heating | 120 | 18 |
| Example 134 | | 0.01 | 60 | 143 | 23 |
| Example 135 | | 0.01 | 100 | 156 | 23 |
| Example 136 | | 0.01 | 180 | 167 | 27 |

(Comparative Examples 21 to 23) Experiment for Heating Raw Aqueous Succinic Acid Solution, and Crystallization Experiment

In a glass flask, 10 L of the test solution obtained in Reference Example 5 was placed. Without addition of an oxidizing agent, the solution was stirred at 60°C, 100°C or 180°C for 2 hours. The APHA values after the heat treatment, and the APHA values of 5 wt% aqueous succinic acid solutions obtained by concentration, crystallization and redissolution in the same manner as in Reference Example 4; are shown in Table 9.

(Comparative Examples 24 to 40) Experiment for Addition of Reducing Agent to Raw Aqueous Succinic Acid Solution, and Crystallization Experiment

In a glass flask, 10 L of the test solution obtained in Reference Example 5 was placed. Each of various reducing agents was added to the test solution, and the resulting mixture was stirred at 25°C (without heating) for 2 hours. The types of the reducing agents added, the APHA values after the treatment with reducing agents, and the APHA values of 5 wt% aqueous succinic acid solutions obtained by concentration, crystallization and redissolution in the same manner as in Reference Example 4; are shown in Table 9.

[Table 9]

| | Reducing agent | Heating temperature (°C) | Concentration of the agent added (%) | Before crystallization | After crystallization |
|---|---|---|---|---|---|
| | | | | APHA | |
| Reference Example 5 | No addition | No heating | 0 | 121 | 29 |
| Comparative Example 18 | No addition | 60 | 0 | 159 | 34 |
| Comparative Example 19 | | 100 | 0 | 181 | 33 |
| Comparative Example 20 | | 180 | 0 | 193 | 37 |
| Comparative Example 21 | NaBH$_4$ | No heating | 1 | 189 | 28 |
| Comparative Example 22 | | | 0.5 | 206 | 34 |
| Comparative Example 23 | | | 0.1 | 203 | 30 |
| Comparative Example 24 | | | 0.01 | 197 | 31 |
| Comparative Example 25 | NaBH(OAc)$_3$ | No heating | 1 | 190 | 35 |
| Comparative Example 26 | | | 0.5 | 207 | 32 |
| Comparative Example 27 | | | 0.1 | 187 | 29 |
| Comparative Example 28 | | | 0.01 | 210 | 36 |
| Comparative Example 29 | BH$_3$·NMe$_3$ | No heating | 1 | 142 | 21 |
| Comparative Example 30 | | | 0.5 | 166 | 26 |
| Comparative Example 31 | | | 0.1 | 176 | 28 |
| Comparative Example 32 | | | 0.01 | 199 | 30 |
| Comparative Example 33 | Hydrazine monohydrate | No heating | 1 | >500 | 33 |
| Comparative Example 34 | | | 0.5 | 343 | 31 |
| Comparative Example 35 | | | 0.1 | 298 | 28 |
| Comparative Example 36 | | | 0.01 | 214 | 29 |

(continued)

|  | Reducing agent | Heating temperature (°C) | Concentration of the agent added (%) | Before crystallization | After crystallization |
|---|---|---|---|---|---|
|  |  |  |  | APHA | |
| Comparative Example 37 | Hydrogen | No heating |  | 178 | 26 |

(Examples 137 to 141) Experiment for Synthesis of 1,4-Butanediol Using Succinic Acid as Raw Material, and Polybutylene Terephthalate Polymerization Experiment

Hydrogenation reaction of succinic acid was carried out according to Examples in JP 4380654 B, to synthesize 1,4-butanediol. More specifically, 254 g of methanol (manufactured by Wako Pure Chemical Industries, Ltd.) and 1.6 g of 95% sulfuric acid (manufactured by Sigma Aldrich) were mixed with 80 g each of the succinic acid crystals obtained in Reference Example 5, Example 72, Example 92, Example 108 and Example 124, and the reaction was allowed to proceed under reflux with stirring for 2 hours. After cooling the reaction solution, 2.9 g of sodium hydrogen carbonate was added thereto, and the resulting mixture was stirred at 60°C for 30 minutes. The mixture was then subjected to distillation at normal pressure, and the distillation residue was filtered and subjected to distillation under reduced pressure, to obtain dimethyl succinate. To the dimethyl succinate, a CuO-ZnO catalyst was added, and the temperature was increased to 230°C for 1 hour in a pressurized reaction vessel in the presence of hydrogen at 5 MPa with stirring. Thereafter, the reaction was allowed to proceed at 230°C under a hydrogen pressure of 15 MPa for 9 hours, and degassing was carried out after cooling. The catalyst was removed from the reaction solution by filtration, and the filtrate was subjected to distillation under reduced pressure, to obtain 49 g of 1,4-butanediol.

With 45.0 g of each synthesized 1,4-butanediol, 94.0 g of terephthalic acid (manufactured by Wako Pure Chemical Industries, Ltd.) was mixed, and 0.07 g of tetra-n-butyl titanate (manufactured by Kanto Chemical Co., Inc.) and 0.06 g of monobutylhydroxytin oxide (manufactured by Tokyo Chemical Industry Co., Ltd.) were added to the resulting mixture as catalysts. The reaction was started in a reactor equipped with a rectifying column at 190°C at 79.9 kPa, and the temperature was increased stepwise while 56.9 g of 1,4-butanediol was gradually added to the reaction mixture (fmal molar concentration: 1,4-butanediol/terephthalic acid=2/1), to obtain an esterified reaction product. To 100 g of this esterified product, 0.06 g of tetra-n-butyl titanate and 0.01 g of phosphoric acid (manufactured by Wako Pure Chemical Industries, Ltd.) as polycondensation catalysts were added, and polycondensation was carried out at 250°C at 67 Pa. The molecular weight, melting point, weight reduction rate upon heating, and APHA of the produced polybutylene terephthalate were measured under the same conditions as those for the polylactic acids of Examples 1 to 68 (as the solvent for measurement of APHA, hexafluoroisopropyl alcohol was used). The results are shown in Table 10.

[Table 10]

|  | Raw material succinic acid crystals | Polybutylene terephthalate | | | |
|---|---|---|---|---|---|
|  |  | Weight average molecular weight ($\times$10000) | Melting point (°C) | Weight reduction rate upon heating (%) | APHA |
| Example 137 | ReferenceExample 5 | 1.81 | 220.3 | 0.56 | 48 |
| Example 138 | Example 72 | 1.94 | 221.1 | 0.41 | 7 |
| Example 139 | Example 92 | 2.45 | 224.5 | 0.27 | 5 |
| Example 140 | Example 108 | 1.98 | 223.0 | 0.36 | 10 |
| Example 141 | Example 124 | 2.17 | 222.7 | 0.34 | 14 |

From the above results of Examples and Comparative Examples, it became clear that addition of an oxidizing agent to a raw aqueous succinic acid solution containing a colored component allows removal of the colored impurity and improvement of properties of polybutylene terephthalate produced by polymerization.

(Reference Example 5) Production of D-Lactic Acid Fermentation Culture Liquid with Transformed Yeast

According to the methods described in the Examples 8 and 10 of WO2010/140602, SU042, which is a D-lactic acid fermentation yeast, was cultured, to obtain a D-lactic acid culture liquid.

(Reference Example 6) Providing Test Solution (Raw Aqueous Lactic Acid Solution) from D-Lactic Acid Fermentation Culture Liquid

Bacterial cells were removed from 30 L of the D-lactic acid culture liquid prepared in Reference Example 5 by filtration through a microfiltration membrane ("Microza", manufactured by Asahi Kasei Corporation), and 95% sulfuric acid (manufactured by Sigma Aldrich) was added to the resulting filtrate until the pH became 2.5, followed by stirring the obtained mixture for 2 hours. The produced calcium sulfate was removed by suction filtration, and the obtained filtrate was passed through a column packed with a strong anion-exchange resin ("DIAION SA10A", manufactured by Mitsubishi Chemical Corporation) in the downflow direction. The resultant was then passed through a column packed with a strong cation-exchange resin ("DIAION SK1B" manufactured by Mitsubishi Chemical Corporation) in the downflow direction. Subsequently, the resultant was filtered through a nanofiltration membrane (4-inch spiral element "SU-610", manufactured by Toray Industries, Inc.), to obtain 28 L of a raw aqueous lactic acid solution. Subsequently, the solution was concentrated to 56 wt% using a thin-film evaporator (manufactured by Tokyo Rikakikai Co., Ltd.), to provide a raw lactic acid test solution. The AHPA value of the raw lactic acid test solution was 49. The raw lactic acid test solution was subjected to distillation at 130°C under a reduced pressure of 133 Pa. The APHA value and the optical purity of the lactic acid obtained by the distillation are shown in Table 11.

In a reaction vessel equipped with a stirrer, 30 g of the obtained lactic acid was heated at 800 Pa at 160°C for 3.5 hours, to obtain oligomers. Subsequently, 24 mg of tin(II) acetate (manufactured by Kanto Chemical Co., Inc.) and 66 mg of methanesulfonic acid (manufactured by Wako Pure Chemical Industries, Ltd.) were added to the oligomers, and the resulting mixture was heated at 500 Pa at 180°C for 7 hours, to obtain a prepolymer. Subsequently, the prepolymer was heated in an oven at 120 °C for 2 hours to allow crystallization. The obtained prepolymer was pulverized using a hammer crusher, and sieved to obtain a powder with an average particle size of 0.1 mm. In the solid phase polymerization step, 10 g of the prepolymer was placed in an oven connected to an oil rotary pump, and heat treatment was performed under reduced pressure. In this treatment, the pressure was 50 Pa, the heating temperatures were: 140°C for 10 hours, 150°C for 10 hours, and 160°C for 20 hours. The obtained polylactic acid was subjected to analysis of the weight average molecular weight by GPC, analysis of the melting point by DSC, analysis of the weight reduction rate upon heating by TG, and measurement of the degree of coloration under the same conditions as in Examples 1 to 68. The results are shown in Table 12.

(Example 142) Experiment for Addition of Ozone Water to Raw Lactic Acid Test Solution, Experiment for Distillation under Reduced Pressure, and Polylactic Acid Polymerization Experiment

In a glass Schlenk flask, 80 mL of the raw lactic acid test solution obtained in Reference Example 6 was weighed. To the test solution, 280 mL of 50 ppm ozone water (manufactured by Unno Giken Co., Ltd.) was added, and the resulting mixture was stirred at room temperature (25°C) for 16 hours. The amount of ozone added at this time was 0.03% as calculated according to (Equation 1). Thereafter, while the test solution was heated from room temperature to 35°C, the test solution was concentrated under a reduced pressure of 20 hPa to a lactic acid concentration of 56%. The obtained concentrate was subjected to distillation at 130°C under a reduced pressure of 133 Pa, to obtain D-lactic acid. The APHA value and the optical purity of the lactic acid after distillation are shown in Table 11. Subsequently, a polymerization test and analysis were carried out in the same manner as in Reference Example 6. The results are shown in Table 12.

(Example 143) Experiment for Addition of Hydrogen Peroxide Solution to Raw Lactic Acid Test Solution, Experiment for Distillation under Reduced Pressure, and Polylactic Acid Polymerization Experiment

In a glass Schlenk flask, 80 mL of the raw lactic acid test solution obtained in Reference Example 6 was weighed. To the test solution, 1.5 mL of 30% hydrogen peroxide solution (manufactured by Wako Pure Chemical Industries, Ltd.) was added, and the resulting mixture was stirred at 120°C for 4 hours. The amount of hydrogen peroxide added at this time was 1% as calculated according to (Equation 1). The resulting product was subjected to distillation at 130°C under a reduced pressure of 133 Pa. The APHA value and the optical purity of the lactic acid obtained by distillation are shown in Table 11. Subsequently, a polymerization test and analysis were carried out in the same manner as in Reference Example 6. The results are shown in Table 12.

(Example 144) Experiment for Addition of Dilute Hydrogen Peroxide Solution to Raw Lactic Acid Test Solution, Experiment for Distillation under Reduced Pressure, and Polylactic Acid Polymerization Experiment

In a glass Schlenk flask, 80 mL of the raw lactic acid test solution obtained in Reference Example 6 was weighed. To the test solution, 280 mL of 50 ppm hydrogen peroxide solution (prepared by diluting 47 μL of 30% hydrogen peroxide solution manufactured by Wako Pure Chemical Industries, Ltd. with 280 mL of distilled water) was added, and the resulting mixture was stirred at 120°C for 4 hours. The amount of hydrogen peroxide added at this time was 0.03% as calculated according to (Equation 1). Subsequently, the test solution was once cooled to room temperature (25°C). Thereafter, while the test solution was heated again to 35°C, the test solution was concentrated under a reduced pressure of 20 hPa to a lactic acid concentration of 56%. The resulting solution was then subjected to distillation at 130°C under a reduced pressure of 133 Pa, to obtain D-lactic acid. The APHA value and the optical purity of the lactic acid obtained by the distillation are shown in Table 11. Subsequently, a polymerization test and analysis were carried out in the same manner as in Reference Example 6. The results are shown in Table 12.

[Table 11]

|  | Oxidizing agent | Concentration of the agent added (%) | Heating temperature (°C) | Before distillation | | After distillation | |
|---|---|---|---|---|---|---|---|
|  |  |  |  | APHA | Optical purity (%e.e.) | APHA | Optical purity (%e.e.) |
| Reference Example 6 | No addition | 0 | No heating | 49 | 100 | 22 | 100 |
| Example 142 | 50ppm Ozone water | 0.03 | 35 | 33 | 100 | 16 | 100 |
| Example 143 | 30% Hydrogen peroxide solution | 1 | 120 | 52 | 100 | 23 | 100 |
| Example 144 | 50ppm Hydrogen peroxide solution | 0.03 | 120 | 58 | 100 | 22 | 100 |

[Table 12]

| Lactic acid | | | Polylactic acid | | | |
|---|---|---|---|---|---|---|
|  | Oxidizing agent | After distillation APHA | Weight average molecular weight (×1000) | Melting point (°C) | Weight reduction rate upon heating (%) | APHA |
| Reference Example 6 | No addition | 22 | 174 | 163.0 | 32 | 17 |
| Example 142 | 50ppm Ozone water | 16 | 220 | 164.8 | 26 | 12 |
| Example 143 | 30% Hydrogen peroxide solution | 23 | 208 | 164.8 | 28 | 12 |
| Example 144 | 50ppm Hydrogen peroxide solution | 22 | 220 | 163.6 | 29 | 15 |

From the results of the above Reference Examples and Examples, it became clear that addition of an oxidizing agent to an aqueous lactic acid solution containing a colored component allows removal of the colored impurity and improvement of properties of polylactic acid produced by polymerization, without decreasing the optical purity of the lactic acid.

INDUSTRIAL APPLICABILITY

Since, by the present invention, colored impurities contained in an organic acid derived from a biomass resource are removed, the obtained organic acid can be suitably used as an industrial chemical product such as a polymer material.

**Claims**

1.  A method for producing an organic acid, said method comprising subjecting an organic acid derived from a biomass resource to oxidation treatment using an oxidizing agent.

2.  The method for producing an organic acid according to claim 1, wherein said oxidizing agent is one or more selected from the group consisting of hydrogen peroxide, tert-butylhydroperoxide, ozone, sodium hypochlorite and sodium chlorite, and aqueous solutions thereof.

3.  The method for producing an organic acid according to claim 1 or 2, wherein said oxidation treatment is combined with heat treatment.

4.  The method for producing an organic acid according to claim 3, wherein said heat treatment is distillation.

5.  The method for producing an organic acid according to any one of claims 1 to 4, wherein said organic acid is an organic acid obtained by fermentation culture of a microorganism(s).

6.  The method for producing an organic acid according to any one of claims 1 to 5, wherein said organic acid is one or more selected from the group consisting of lactic acid, hydroxybutyric acid, 3-hydroxypropionic acid, itaconic acid, glycolic acid, adipic acid, muconic acid, acrylic acid, succinic acid, sebacic acid, 2,5-furandicarboxylic acid and terephthalic acid.

7.  A method for producing an organic acid polymer, said method comprising polymerizing as a raw material an organic acid obtained by the method for producing an organic acid according to any one of claims 1 to 6.

| | INTERNATIONAL SEARCH REPORT | International application No. |
|---|---|---|
| | | PCT/JP2012/068427 |

A. CLASSIFICATION OF SUBJECT MATTER
*C12P7/40*(2006.01)i, *C07C51/487*(2006.01)i, *C07C59/08*(2006.01)i, *C08G63/78*
(2006.01)i

According to International Patent Classification (IPC) or to both national classification and IPC

B. FIELDS SEARCHED

Minimum documentation searched (classification system followed by classification symbols)
C12P7/40, C07C51/487, C07C59/08, C08G63/78

Documentation searched other than minimum documentation to the extent that such documents are included in the fields searched

Electronic data base consulted during the international search (name of data base and, where practicable, search terms used)
CAPLUS/BIOSIS/MEDLINE/WPIDS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 2010-539911 A (Uhde GmbH), 24 December 2010 (24.12.2010), & US 2010/0273224 A1 & EP 2195441 A2 & WO 2009/040095 A2 | 1-7 |
| Y | JP 2009-201506 A (Toray Industries, Inc.), 10 September 2009 (10.09.2009), (Family: none) | 1-7 |
| Y | WO 2010/074222 A1 (Toray Industries, Inc.), 01 July 2010 (01.07.2010), & EP 2374895 A1 & WO 2010/074222 A1 | 1-7 |
| Y | JP 2009-142265 A (Toray Industries, Inc.), 02 July 2009 (02.07.2009), (Family: none) | 1-7 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 13 August, 2012 (13.08.12) | Date of mailing of the international search report 21 August, 2012 (21.08.12) |
|---|---|
| Name and mailing address of the ISA/ Japanese Patent Office | Authorized officer |
| Facsimile No. | Telephone No. |

Form PCT/ISA/210 (second sheet) (July 2009)

24

**INTERNATIONAL SEARCH REPORT**

| International application No. |
| --- |
| PCT/JP2012/068427 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | WO 2010/035837 A1  (Mitsubishi Chemical Corp.), 01 April 2010 (01.04.2010), & JP 2010-100617 A | 1-7 |
| Y | JP 2002-510747 A  (Metabolix, Inc.), 09 April 2002 (09.04.2002), & US 2001/0006802 A1    & EP 1070135 A1 & WO 1999/051760 A1 | 1-7 |
| Y | JP 2008-504035 A  (The Procter & Gamble Co.), 14 February 2008 (14.02.2008), & US 2005/0287654 A1    & EP 1781721 A1 & WO 2006/004895 A1 | 1-7 |
| Y | JP 2008-54541 A  (Kaneka Corp.), 13 March 2008 (13.03.2008), (Family: none) | 1-7 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3880175 B
- JP 2008029329 A
- JP 6038775 A
- JP 4490628 B
- JP 1913914 B
- JP 2008056654 A
- JP 4380654 B
- WO 2011094131 A
- JP 2011084540 A
- US 5487987 B
- JP 2004075932 A
- WO 2009099044 A
- WO 2010140602 A

**Non-patent literature cited in the description**

- *Enzyme and Microbial Technology,* 2000, vol. 27, 205
- *Science,* 2010, vol. 330, 1222
- Polyamide Resin Handbook. Nikkan Kogyo Shimbun, Ltd, 1998